(19) Europäisches Patentamt
European Patent Office
Office européen des brevets

(11) **EP 2 532 351 B1**

(12) **EUROPEAN PATENT SPECIFICATION**

(45) Date of publication and mention of the grant of the patent:
**08.07.2015 Bulletin 2015/28**

(21) Application number: **11739761.2**

(22) Date of filing: **02.02.2011**

(51) Int Cl.:
*A61K 31/688* (2006.01)    *A61P 3/04* (2006.01)
*A61P 3/06* (2006.01)    *A61P 43/00* (2006.01)
*A23L 1/30* (2006.01)

(86) International application number:
**PCT/JP2011/052098**

(87) International publication number:
**WO 2011/096413 (11.08.2011 Gazette 2011/32)**

(54) **AGENT FOR IMPROVING MOTILITY FUNCTION**

MITTEL ZUR VERBESSERUNG VON BEWEGLICHKEITSFUNKTIONEN

AGENT POUR AMÉLIORER LA MOTILITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.02.2010 JP 2010022223**
**03.02.2010 JP 2010022221**

(43) Date of publication of application:
**12.12.2012 Bulletin 2012/50**

(73) Proprietor: **Kao Corporation**
**Chuo-ku**
**Tokyo 103-8210 (JP)**

(72) Inventors:
• **HARAMIZU, Satoshi**
**Haga-gun**
**Tochigi 321-3497 (JP)**
• **OTA, Noriyasu**
**Haga-gun**
**Tochigi 321-3497 (JP)**
• **HASHIZUME, Kohjiro**
**Haga-gun**
**Tochigi 321-3497 (JP)**

• **MURASE, Takatoshi**
**Haga-gun**
**Tochigi 321-3497 (JP)**

(74) Representative: **Vossius & Partner**
**Patentanwälte Rechtsanwälte mbB**
**Siebertstrasse 3**
**81675 München (DE)**

(56) References cited:
WO-A1-2008/081934    JP-A- 2000 350 563
JP-A- 2002 226 394    JP-A- 2006 516 568
JP-A- 2007 161 703    JP-A- 2010 059 155
US-A1- 2004 043 013    US-A1- 2009 253 658

• **DANIELI-BETTO DANIELA ET AL: "Sphingosine 1-phosphate protects mouse extensor digitorum longus skeletal muscle during fatigue", AMERICAN JOURNAL OF PHYSIOLOGY - CELL PHYSIOLOGY, vol. 288, no. 6, June 2005 (2005-06), pages C1367-C1373, XP002695869,**
• **DOBRZYN A ET AL.: 'Ceramides and sphingomyelins in skeletal muscles of the rat: content and composition. Effect of prolonged exercise.' AM J PHYSIOL ENDOCRINOL METAB vol. 282, no. 2, 2002, pages E277 - E285**

SEQUENCE LISTING

    <110> Kao corporation

    <120> Motor function improver

    <130> U2345 EP S3

    <140> EP 11 73 9761.2
    <141> 2011-02-02

    <150> JP 2010-022221
    <151> 2010-02-03

    <150> JP 2010-022223
    <151> 2010-02-03

    <160> 4

    <170> PatentIn version 3.1

    <210> 1
    <211> 20

<212> DNA
<213> Artificial sequence

<220>
<223> Oligonucleotide from 36B4, as forward PCR primer

<400> 1
gacatcacag agcaggccct        20

<210> 2
<211> 20
<212> DNA
<213> Artificial sequence

<220>
<223> Oligonucleotide from 36B4, as reverse PCR primer

<400> 2
tctccacaga caatgccagg 20

<210> 3
<211> 20
<212> DNA
<213> Artificial sequence

<220>
<223> Oligonucleotide from PGC-1\203¿, as forward PCR primer

<400> 3
ccgagaattc atggagcaat        20

<210> 4
<211> 20
<212> DNA
<213> Artificial sequence

<220>
<223> Oligonucleotide from PGC-1\203¿, as reverse PCR primer

<400> 4
tttctgtggg tttggtgtga        20

**Description**

Field of the Invention

The present invention relates to a sphingomyelin for use in a method for suppressing muscle strength deterioration. Also disclosed are a motor function improver, a mitochondrial function improver, an energy consumption promoter, and a lipid combustion promoter which exhibit a motor function improving effect.

Background of the Invention

In general, for improving motor function such as endurance or muscle strength, exercise training and well-balanced nutrition are thought to be important. Recently, sports lovers and athletes have attempted to employ not only training but also alimentation with supplements and the like to improve their muscle strength more efficiently (Patent Document 1). However, there is a concern that training with excess intake of certain proteins or amino acids may have an adverse effect on the kidney function and the like (Non-Patent Document 1).

In persons other than sports lovers or athletes, there are also concerns about: decrease in skeletal muscle or deterioration in muscle strength due to insufficient internal supply of nutritional components through unreasonable restriction of diets; deterioration in motor functions such as muscle strength and endurance due to muscle weakness associated with aging or disuse of muscle; and fatigue associated with deterioration in motor function.

Therefore, efficient motor function improvement technologies are being desired by not only sports lovers and athletes who aim to improve their performance but also ordinary persons.

From such a viewpoint, components having a motor function improving effect have been searched for. Consequently, for example, endurance improving action of tea catechin (Patent Document 2), muscle strength improving action of polymeric polyphenol derived from a fruit (Patent Document 3), phytic acid (Patent Document 4), and the like have been reported.

Furthermore, in recent years, the probability of an effect of a phospholipid on the motor function has been clarified, and for example, lactate accumulation suppressing action during exercise of phosphatidylcholine (Non-Patent Document 2), endurance improving action of phosphatidylserine (Non-Patent Document 3), and the like have been reported.

On the other hand, our life activities are supported by ATP produced by linkageof various physical/chemical processes called metabolism. Mitochondria play a central role in energy metabolism and supply ATP by β-oxidation of a fatty acid or oxidative phosphorylation by an electron transport system.

Oxygen consumption which reflects energy consumption in a living organism is characterized by being high in the skeletal muscle, liver, or heart. The fact corresponds to that the mitochondria are distributed at a high level in the heart muscle, liver, and skeletal muscle, indicating that the mitochondria play an important role in energy metabolism. It is known that 90% or more of oxygen consumption in a living organism are carried out in the mitochondria.

In recent years, it has been clarified that dysfunction of the mitochondria is closely related to lifestyle-related diseases, aging-related diseases, and the like. Reduction in energy metabolism due to aging is known to relate to decrease in the mitochondrial function such as a mutation or a damage of mitochondrial DNA (Non-Patent Document 4).

Decrease in the mitochondrial function may cause an imbalance of energy intake and energy consumption via reduction in energy metabolism, and hence may cause lifestyle-related diseases (Non-Patent Document 5). Therefore, enhancement of the energy metabolism by maintaining/improving the mitochondrial function may lead to prevention/improvement of lifestyle-related diseases and may contribute to quality-of-life (QOL).

Exercise is known to be a method of increasing the amount of mitochondria in muscle (Non-Patent Document 6). Therefore, the exercise may increase energy consumption in a living organism via an increase in mitochondria in muscle. However, although importance of exercise is widely recognized at the present day, in reality, it is difficult to carry out exercises regularly. A method of increasing energy consumption by promoting energy metabolism more effectively has been desired.

From such a viewpoint, components for enhancing mitochondrial function and energy metabolism have been searched for. Consequently, for example, caffeine, capsaicin and the like having sympathetic nervous activating action have been reported as components for promoting energy metabolism (Non-Patent Documents 7 and 8). However, caffeine and capsaicin are unsatisfactory because their practical applications are limited from the viewpoints of safety, irritant property and the like. Further examples of the components having energy metabolism promoting action include capsinoid-containing compositions (Patent Document 5) and flavans or flavanones (Patent Document 6).

Furthermore, in recent years, it has been reported that capsiate, which is a less-pungent, low-irritant capsaicin analog, has energy metabolism promoting action (Non-Patent Document 9).

Furthermore, it has been found that tea catechin has an action of suppressing reduction in energy metabolism and deterioration of the mitochondrial function due to aging (Patent Document 7). In addition, as components having mitochondrial function activating action, there are given, for example, a benzimidazole derivative or a salt thereof (Patent Document 8) and 1,2-ethanediol or a salt thereof (Patent Document 9). However, few components for enhancing energy metabolism and mitochondrial function other than the foregoing are known.

It is known that sphingomyelin is a compound having a structure in which phosphocholine is bound to a ceramide skeleton including a sphingoid base and a fatty acid, and is present at a high level in the brain and nerve tissue in a living organism. In recent years, studies on physiological functions of the sphingomyelin have been made, and the sphingomyelin is known to have physiological functions such as a promoting effect on maturing or development of the digestive tract (Patent Document 10), a learning ability improving effect (Patent Document 11), and a sialomucin secretion promoting effect (Patent Document 12). In addition, it has been reported that the sphingomyelin relates to activation of muscle satellite cells (Non-Patent Document 10) and has an anti-inflammatory action (Non-Patent Document 11).

Moreover, it is known that the sphingomyelin can be used, for example, as an agent for improving lipid digestive and absorptive function of intestinal tract (Patent Document 13) or an agent for treatment of bowel movement dysfunction (Patent Document 14).

In addition, JP 2002-226394 discloses a lipid-metabolism improvement composition comprising sphingomyelin as the main component for treating or preventing lifestyle-related diseases. JP 2000-350563 discloses a nutrition composition for infants comprising sphingomyelin. WO 2008/081934 discloses an agent for facilitating the development of the brain in an infant, which comprises an effective amount of a milk-derived phospholipid or a sphingomyelin. US 2009/253658 discloses a fat accumulation inhibitor for a fat cell, which comprises a milk-derived phospholipid as an active ingredient. US 2004/043013 discloses a composition comprising sphingomyelin for muscle fatigue due to exercise. Danieli-Betto et al., American Journal of Physiology - Cell Physiology 288, No 6, pages C1367-C1373, discloses that exogeneous administration of sphingosine and sphingosine 1 phosphate causes a reduction of tension decline during fatigue.

However, effects of the sphingomyelin on motor function such as muscle strength or endurance and further on mitochondrial function and energy metabolism have not been known heretofore.

Prior Art Document

Patent Document

[Patent Document 1] JP-A-2002-065212
[Patent Document 2] JP-A-2005-89384
[Patent Document 3] WO 2005/074962 pamphlet
[Patent Document 4] JP-A-2009-107987
[Patent Document 5] JP-A-2004-149494
[Patent Document 6] JP-A-2007-314446
[Patent Document 7] JP-A-2008-63318
[Patent Document 8] JP-A-2004-67629
[Patent Document 9] JP-A-2002-322058
[Patent Document 10] JP-A-2000-250563
[Patent Document 11] JP-A-2007-246404
[Patent Document 12] JP-A-2007-112793
[Patent Document 13] JP-A-11-269074
[Patent Document 14] JP-A-2003-252765

Non-Patent Document

[Non-Patent Document 1] Anderson, JAMA, 223, 1973 [Non-Patent Document 2] Von Allworden, Phospholipids, AOCS Press, 1995
[Non-Patent Document 3] Kingsley, Med Sci Sports Exerc, 38, 2006
[Non-Patent Document 4] Iwanamikouza: Gendaiigaku no kiso, 1999 12(2): 55-58.
[Non-Patent Document 5] Ritz P. Diabetes Metab. 2005 2: 5S67-5S73.
[Non-Patent Document 6] Holloszy JO. J. Physiol. Pharmacol. 2008 59: 5-18.
[Non-Patent Document 7] Dulloo AG. Am J Clin Nutr. 1989 49 (1) : 44-50.
[Non-Patent Document 8] Kawada T. Proc Soc Exp Biol Med. 1986 183 (2) : 250-6.

[Non-Patent Document 9] Ohnuki K. Biosci Biotechnol Biochem. 2001 65(12): 2735-40.
[Non-Patent Document 10] Nagata Y. J Histochem Cytochem. 2006 54(4): 375-384.
[Non-Patent Document 11] Furuya H. Int J Vitam Nutr Res. 2008 78(1): 41-49.

Summary of the Invention

The invention relates to the embodiments as defined in the claims. The present invention provides a sphingomyelin for use in a method for suppressing muscle strength deterioration. It also relates to the sphingomyelin of the invention for use in a method for treating or preventing locomotive syndrome. In addition, the invention relates to the sphingomyelin for use in a method for treating or preventing sarcopenia.

Moreover, the present disclosure relates to the following items.

(1) A motor function improver, including a sphingomyelin as an active ingredient.
(2) An endurance improver, including a sphingomyelin as an active ingredient.
(3) A muscle strength improver, including a sphingomyelin as an active ingredient.
(4) A muscle strength deterioration suppressor, including a sphingomyelin as an active ingredient.
(5) An anti-fatigue agent, including a sphingomyelin as an active ingredient.
(6) A mitochondrial function improver, including a sphingomyelin as an active ingredient.
(7) An energy consumption promoter, including a sphingomyelin as an active ingredient.
(8) A lipid combustion promoter, including a sphingomyelin as an active ingredient.

Brief Description of the Drawings

[FIG. 1] A graph showing a transition of swimming endurance. Ex represents a normal diet and exercise group, and SPM represents a 0.25% sphingomyelin diet and exercise group.
[FIG. 2] A graph showing muscle strength of the isolated soleus muscle and extensor digitorum longus muscle. Cont represents a normal diet group, Ex represents a normal diet and exercise group, and SPM represents a 0.25% sphingomyelin diet and exercise group.
[FIG. 3] A graph showing muscle strength of the isolated soleus muscle. Normal represents a normal diet and untreated (non-tail suspension) group, Cont represents a normal diet and tail suspension group, and SPM represents a 0.25% sphingomyelin diet and tail suspension group. # represents a significant difference relative to Normal group.

Detailed Description of the Invention

The present invention relates to providing a material which is commonly consumed in diet with high safety and to be blended in a drug, a quasi drug, a food, and a feed that exhibit an excellent motor function improving action, mitochondrial function improving action, energy consumption promoting action, and lipid combustion promoting action.

The present inventors have searched for components which are effective for improving motor function, improving mitochondrial function, promoting energy consumption, and promoting lipid combustion, and, as a result, have found that a sphingomyelin has an effect of a motor function improving action, an endurance improving action, a muscle strength improving action, a muscle strength deterioration suppressing action, a mitochondrial function improving action, an energy consumption promoting action, and a lipid combustion promoting action, and that the component is useful as an active ingredient in drugs, quasi drugs, foods, drinks, and feeds that can exhibit such effects.

The muscle strength deterioration suppressor according to the present invention is useful as material to be blended as active ingredients in foods, drugs, quasi drugs, or feeds for improvement in motor function, improvement in endurance, anti-fatigue, improvement in muscle strength, or suppression of muscle strength deterioration in exercises as well as daily activity including labor for persons in all ages including aged persons.

Meanwhile, the mitochondrial function improver, energy consumption promoter, and lipid combustion promoter of the present disclosure are useful as materials to be blended as active ingredients in foods, drugs, quasi drugs, or feeds for preventing or improving deterioration of mitochondrial function or energy metabolism.

The sphingomyelin which can be used in the present invention is not particularly limited, and examples thereof include chemically synthesized sphingomyelins and naturally-derived sphingomyelins.

For example, as a chemical synthesis method for sphingomyelin, there are known methods for converting a ceramide into a sphingomyelin by introducing a phosphocholine into the hydroxyl group at position 1 of the ceramide via 1) a phosphoramidite (Weis, Chem Phys Lip, 3, 1999), 2) a cyclic phosphate (Dong, Tetrahedron Lett, 5291, 1991), or 3) a cyclic phosphite (Byun, J Org Chem, 6495, 1994).

Furthermore, a highly pure sphingomyelin can be obtained by purifying a milk fat globule membrane component obtained from cow milk by techniques such as dialysis, ammonium sulfate fractionation, gel filtration, isoelectric precipitation, ionexchange chromatography, and solvent fractionation (Sanchez-Juanes, Int Dairy J, 273, 2009).

Moreover, the sphingomyelin may be a commercially available product. Examples of the commercially available product include "cow milk-derived sphingomyelin: NM-70" and "yolk-derived sphingomyelin: NM-10" produced by NOF CORPORATION.

As described in the below-mentioned Examples, since the sphingomyelin significantly prolonged swimming time and significantly increased muscle strength of the soleus muscle in mice, the sphingomyelin has an endurance improving action, a muscle strength improving action, and an anti-fatigue action proved by such improvement in motor function. Furthermore, since the sphingomyelin significantly suppresses muscle strength deterioration in mice subjected to a muscle non-use (tail suspension) treatment to thereby deteriorate muscle strength, the sphingomyelin has a muscle strength deterioration suppressing action.

The endurance and muscle strength are representative motor functions for taking physical actions. Therefore, the sphingomyelin can be used in methods for improvement in motor function, improvement in endurance, improvement in muscle strength, and anti-fatigue in exercises as well as broadly-defined exercises including daily performance and labor, or a method for suppressing muscle strength deterioration, by administration or intake of the sphingomyelin in animals including humans.

In the present invention, motor function improvement refers to not only improving/ameliorating the motor ability of athletes and sports lovers, but also improving/ameliorating the physical activity level in persons whose motor organ function is reduced due to the onset of muscle atrophy, locomotive syndrome and the like, via improvement/amelioration in muscle strength, endurance, or the like. Note here that the locomotive syndrome refers to a syndrome in motor organ, which is thought to be caused by motor organ dysfunction related to diseases in the motor organ itself or aging. Examples of the motor organ dysfunctions related to aging include muscle strength deterioration, endurance deterioration, prolonged reaction time, deterioration in movement speed, deterioration in skillness, bathyhypesthesia, and balance ability deterioration. Furthermore, insufficient exercise in addition to the aging may cause deterioration in entire motor organ function in addition to the above-mentioned deterioration in muscle strength or balance ability, thus causing problems such as a tendency to fall. Therefore, the sphingomyelin of the present invention can be used to treat locomotive syndrome or sarcopenia, specifically, can be used to reduce the risk of developing, prevent or improve locomotive syndrome, and reduce the risk of developing, prevent or improve sarcopenia.

Furthermore, as described in the below-mentioned Examples, the sphingomyelin has a mitochondrial function improving action, energy consumption promoting action, and lipid combustion promoting action.

Therefore, the sphingomyelin can be used in a method for improving mitochondrial function, promoting energy consumption, and promoting lipid combustion by administration or intake of the sphingomyelin in animals including humans.

Mitochondria are present in many cells in a living organism and play a particularly important role in ATP production by an oxidative phosphorylation reaction. That is to say, in the present invention, the mitochondrial function means that energy for cell survival/activity is obtained from nutrients such as carbohydrates and lipids. Furthermore, since it is thought that mitochondrial dysfunction is closely related to lifestyle-related diseases, aging-related diseases, and the like, the sphingomyelin of the present disclosure can be used for preventing/improving insulin resistance-related diseases such as obesity and diabetes, and prostration and fatigue due to aging and inaction.

Furthermore, in the present disclosure, the energy consumption means that nutrients (energy sources) are metabolized in each tissue of a living organism and converted into chemical energy or thermal energy, and the energy consumption level is calculated from the level of oxygen consumed in the process and refers to a macro physicochemical energy production level in each individual. That is to say, the energy consumption promoting action refers to an action to increase the energy consumption level defined as above. In addition, the lipid combustion means that a fatty acid is metabolized in each tissue in a living organism and converted into chemical energy or thermal energy.

The lipid combustion level is calculated from the level of oxygen consumed and the level of carbon dioxide emitted in the oxidative metabolism process by using, for example, the following equation (i) of Peronnet et al. (Peronnet et al., Can J Sport Sci. 1991 16:23-29), and refers to a level of production of energy derived from lipids in each individual. That is to say, the lipid combustion promoting action refers to an action of increasing the lipid combustion level defined as above.

$$\text{Lipid combustion level} = 1.695 \times (1 - 1.701/1.695 \times \text{respiratory quotient}) \times \text{oxygen consumption level} \quad (i)$$

(provided, respiratory quotient = carbon-dioxide emission level/oxygen consumption level)

Furthermore, the sphingomyelin can be used as a motor function improver, an endurance improver, a muscle strength improver, an anti-fatigue agent, and a muscle strength deterioration suppressor (hereinafter, referred to as "motor function improver and the like"), or as a mitochondrial function improver, an energy consumption promoter, and a lipid combustion promoter (hereinafter, referred to as "mitochondrial function improver and the like"), and can further be used for production of the motor function improver and the like and the mitochondrial function improver and the like. At this time, for the motor function improver and the like, or for the mitochondrial function improver and the like, the sphingomyelin may be used alone, or in combination with appropriately selected additives such as a carrier which are acceptable to the below-mentioned target products to incorporate therein, if necessary. Note here that the improver and the like can be produced by conventional methods depending upon the target products to incorporate therein.

The motor function improver and the like according to the present invention can be used as active ingredients to be blended in drugs, quasi drugs, foods, or feeds for humans or animals, which exhibit a motor function improving effect, an endurance improving effect, a muscle strength improving effect, an anti-fatigue effect, or a muscle strength deterioration suppressing effect. Furthermore, the motor function improver and the like according to the present invention have the concept of achieving endurance improvement, muscle strength improvement, anti-fatigue, or muscle strength deterioration suppression in persons with insufficient exercise, middle aged and older persons, persons who need bed rest, or athletes and sports lovers, and the motor function improver and the like can be applied for foods, functional foods, patient foods, foods for specified health, to which the concept is presented as needed.

Furthermore, the mitochondrial function improver and the like according to the present invention can be administered to humans and animals and can be used as active ingredients to be blended in various foods, drinks, drugs, pet foods, and the like. The mitochondrial function improver and the like according to the present invention to be blended in foods have the concept of achieving physiological functions such as improvement in mitochondrial function or promotion of energy consumption, and prevention, improvement, or reduction in risk of development of lifestyle-related diseases, and the mitochondrial function improver and the like can be applied for foods and drinks, functional foods and drinks, patient foods and drinks, foods for specified health, and the like, to which the concept is presented as needed.

The forms of administration of the motor function improver and the like and the mitochondrial function improver and the like according to the present invention used for active ingredients of drugs or quasi drugs include, for example, oral administration such as tablets, capsules, granules, powders, and syrups, and parenteral administration such as injections, suppositories, inhalation drugs, transdermal systems, and external preparations. Furthermore, when preparations in such various dosage forms are prepared, the motor function improver and the like and the mitochondrial function improver and the like according to the present invention can be used alone or appropriately in combination with a pharmaceutically acceptable excipient, binder, extender, disintegrant, surfactant, lubricant, dispersing agent, buffering agent, preservative, corrigent, flavor, coating agent, carrier, diluent, or the like. Among these forms of administration, oral administration is preferred. A liquid preparation for oral administration can be prepared by a conventional method by addition of a corrigent, a buffering agent, a stabilizing agent, and the like.

When the motor function improver and the like and the mitochondrial function improver and the like according to the present invention are used for active ingredients of foods, they can be used in the forms of various foods such as foods and drinks and nourishing foods. Examples of such foods include cow milk, processed milk, milk beverages, yogurt, refreshing beverages, tea beverages, coffee beverages, fruit juice beverages, carbonated drink, juice, jelly, wafer, biscuits, bread, noodles, and sausage. In addition, the examples include a nutrient supplying composition having the same forms as the above-mentioned oral administration preparation (tablets, capsules, syrups, and the like).

When various forms of foods are prepared, the motor function improver and the like or the mitochondrial function improver and the like according to the present invention may be used alone or appropriately in combination with other food materials or a solvent, a softener, an oil, an emulsifying agent, an antiseptic, a flavor, a stabilizing agent, a colorant, an antioxidant, a moisturizing agent, a thickening agent, and active ingredients other than the sphingomyelin.

The motor function improver and the like according to the present invention may be blended in motor function improving foods, endurance improving foods, anti-fatigue foods, muscle strength improving foods, pet foods, and the like.

Furthermore, the motor function improver and the like according to the present invention can be blended as a nutritional composition such as an enteral nutrient for aged persons or patients who need bed rest, who have difficulty in taking an adequate amount of nutrients.

Furthermore, foods containing the mitochondrial function improver and the like according to the present invention can be used as foods for improvement in mitochondrial function, foods for promotion of energy consumption, or foods for promotion of lipid combustion.

Furthermore, in the case where the motor function improver and the like or the mitochondrial function improver and the like according to the present invention are used as active ingredients of feeds, they may be used widely in feeds for all livestock animals, and examples of the feeds include: feeds for livestock animals used for cattle, swine, poultry, sheep, horses, and goats; feeds for small animals used for rabbits, rats, and mice; feeds for fish and shellfish used for tuna, eel, sea bream, yellowtail, and shrimp; and pet foods used for dogs, cats, birds, and squirrels.

In addition to the motor function improver and the like or the mitochondrial function improver and the like according to the present invention, a general feed raw material such as a meat, a protein, a cereal, a bran, a lees, a saccharide, a vegetable, a vitamin, or a mineral, or a solvent, a softener, an oil, an emulsifying agent, an antiseptic, a flavor, a stabilizing agent, a colorant, an antioxidant, a moisturizing agent, a thickening agent, or the like may be appropriately blended in the feeds to produce the feeds by a conventional method.

The content of the sphingomyelin (in terms of dry matter) with respect to beverages containing the motor function improver and the like or the mitochondrial function improver and the like according to the present invention, such as milk beverages, refreshing beverages, and tea beverages is generally 0.0001 to 1.0% by mass, preferably 0.001 to 0.5% by mass, and more preferably 0.01 to 0.2% by mass.

In the case of foods or feeds, which are other than the beverages containing the motor function improver and the like or the mitochondrial function improver and the like according to the present invention, or drugs including oral solid preparations such as tablets, granules, and capsules, or oral liquid preparations such as internal liquids and syrups, the content of the sphingomyelin (in terms of dry matter) is generally 0.002 to 50% by mass, preferably 0.02 to 25% by mass, and more preferably 0.2 to 10% by mass. Note here that the state of the sphingomyelin is not particularly limited and may be dissolved or dispersed.

The amount of intake of the motor function improver and the like or the mitochondrial function improver and the like according to the present invention differs depending on the dosage forms or uses, but the content of the sphingomyelin in drugs, foods, drinks, or feeds may be adjusted such that the daily dosage for an adult individual of the sphingomyelin is preferably set at from 0.1 to 1000 mg/60 kg body weight, more preferably at from 1 to 250 mg/60 kg body weight, and even more preferably at from 5 to 100 mg/60 kg body weight.

Furthermore, the motor function improver and the like or the mitochondrial function improver and the like can be administered in an arbitrary administration/intake regimen, and administration/intake is preferably separated once to several times per day.

The motor function improver and the like according to the present invention are preferably administered or taken during physical activity although the timing is not particularly limited. In particular, exercise is preferably combined with the administration or intake. When exercise is combined, the motor function improver and the like according to the present invention are preferably taken within from one hour before the exercise to one hour after the exercise. The exercises to be combined include exercises with strength capable of suppressing deterioration in muscle strength or with strength capable of improving muscle strength when such exercises are continued.

Furthermore, the above-mentioned preparation is administered or taken preferably three days or more per week, more preferably five days or more per week, and even more preferably every day. Furthermore, the duration of administration or intake is preferably two weeks or longer and more preferably four weeks or longer.

Subjects of administration or intake are not particularly limited as long as they are in need thereof. However, since the motor function improver and the like according to the present invention can improve/ameliorate the motor function, they are administered to or taken effectively by, in particular, sports lovers or athletes, persons with locomotive syndrome, persons with sarcopenia, persons with nervous/muscle diseases (inflammatory muscle diseases, myopathy associated with medical diseases, muscular dystrophy, congenital myopathy, mitochondrial encephalomyopathy, glycogen storage disease, and the like), persons with insufficient exercise, persons who need bed rest, and persons who undergo rehabilitation training after surgical/medical diseases.

Furthermore, the mitochondrial function improver and the like are preferably administered or taken during daily activity, for example, during housekeeping or work or when commuting to school or office although the timing is not particularly limited. Furthermore, the above-mentioned preparation is administered or taken preferably three days or more per week, more preferably five days or more per week, and even more preferably every day. Furthermore, the duration of administration or intake is preferably two weeks or longer and more preferably four weeks or longer.

Subjects of administration or intake are not particularly limited as long as they are in need thereof. However, since the mitochondrial function improver and the like according to the present invention can improve the mitochondrial function, promote energy consumption, and promote lipid combustion, they are administered to or taken effectively by, in particular, obese persons, persons with insulin resistance such as diabetic persons, aged persons, and persons with other mitochondrial dysfunction-related diseases (mitochondrial diseases such as Fukuhara disease, Leigh's encephalopathy, mitochondrial diabetes, Leber disease, Pearson disease, Kearns-Sayre syndrome, and stroke-like episode syndrome, fatty liver diseases, and the like).

Examples

Production Example 1: Preparation of sphingomyelin

4000 mL of acetone were added to 201 g of a milk phospholipid PC-500 (available from Fonterra Japan, containing 8.8% by mass (hereinafter, referred to as "%") of a sphingomyelin), and the milk phospholipid was dispersed using a homomixer (TK autohomomixer, produced by Tokusyukika Kogyo Co. Ltd.) under ice cooling. After that, the mixture was centrifuged to remove an acetone-soluble fraction (neutral lipid). To the resultant acetone-insoluble fraction A, 800 mL of chloroform, 400 mL of methanol, and 300 mL of water were added to carry out liquid-liquid extraction, and the chloroform layer was collected. The chloroform layer was concentrated under reduced pressure to obtain 154 g of a chloroform fraction A.

28.05 g of potassium hydroxide and 1000 mL of methanol were added to the resultant chloroform fraction A, and the mixture was stirred under nitrogen at 37°C for 15 hours to carry out hydrolysis. After completion of the reaction, 2000 mL of chloroform and 750 mL of water were added thereto to carry out liquid-liquid distribution, and the chloroform layer was collected. The chloroform layer was concentrated under reduced pressure and neutralized with 15 mL of acetic acid, and 400 mL of chloroform, 200 mL of methanol, and 150 mL of water were added thereto to carry out liquid-liquid extraction again. The chloroform layer was collected and concentrated under reduced pressure to obtain 90 g of a chloroform fraction B.

1200 mL of acetone were added to the resultant chloroform fraction B, and the fraction was dispersed using a homomixer (TK autohomomixer, produced by Tokusyukika Kogyo Co. Ltd.) under ice cooling. After that, the mixture was centrifuged to remove an acetone-soluble fraction (free fatty acids). The same procedure was repeated further twice to obtain 44 g of an acetone-insoluble fraction B.

To the resultant acetone-insoluble fraction B, 400 mL of hexane, 400 mL of methanol, 150 mL of water, and 50 mL of a 28% aqueous ammonia solution were added to carry out liquid-liquid extraction, and the hexane layer was collected. 400 mL of methanol, 150 mL of water, and 50 mL of the 28% aqueous ammonia solution were further added to the hexane layer to carry out washing. The resultant hexane layer was concentrated under reduced pressure to obtain 27 g of a hexane fraction.

22 g of the resultant hexane fraction were purified by silica gel column chromatography. That is to say, the hexane fraction in chloroform solution was adsorbed to 1 kg of silica gel (Silica gel 60 produced by Merck), and contaminants were eluted with a chloroform/methanol mixed solvent, followed by elution with 21 L of methanol to obtain 16 g of a purified sphingomyelin fraction (yield: 8%).

Test Example 1: Effects of sphingomyelin on improvement in endurance and improvement in muscle strength

<Method>

The sphingomyelin was extracted from a milk phospholipid (PC-500, Fonterra Japan) according to the above-mentioned method of Production Example 1.

After preliminary rearing for one week, nine-week old BALB/c male mice (Charles River Laboratories Japan Inc.) were classified into three groups (a Cont group, an Ex group, and an SPM group) (eight mice in each group) such that each group had the same body weight and swimming endurance (determined by measuring a limit swimming time by using a flowing water pool for mice (Kyodai Matsumoto type flowing water tank for measuring an amount of exercise: length × width × depth = 90×45×45 cm, water depth: 38 cm, water temperature: 34°C (Matsumoto, J Appl Physiol. 81: 1843-1849, 1996)) by the below-mentioned method).

After grouping, the mice of the Cont group and the Ex group were fed with a control feed (10% lipid, 20% casein, 55.5% potato starch, 8.1% cellulose, 0.2% methionine, 2.2% vitamin (product name : Vitamin mix AIN-76, Oriental Bioservice, Inc.), and 4% mineral (product name: Mineral mix AIN-76, Oriental Bioservice, Inc.)), and the mice of the SPM group were fed with a test feed containing the sphingomyelin prepared in Production Example 1 (10% lipid, 20% casein, 55.25% potato starch, 8.1% cellulose, 0.2% methionine, 2.2% vitamin, 4% mineral, and 0.25% sphingomyelin) each for 13 weeks.

During the feeding period, the limit swimming time was measured for the mice of the Ex group and the SPM group using the flowing water pool for mice once a week. The limit swimming time was defined as a time from a time of beginning of swimming to a time at which a mouse cannot come to the water surface for seven seconds to breathe at a flow rate of 7 L/min. Note here that during the period, in order to habituate the mice to the exercise, swimming training (6 L/min, 30 min) was given twice a week.

After 13 weeks of rearing, the mice were subjected to anatomy, muscle strength of isolated soleus muscle and extensor digitorum longus muscle was measured. Muscle strength of the isolated muscle was measured according to a method by Cannon et al. (Biomed Sci Instrum, 2005). That is to say, the soleus muscle and extensor digitorum longus muscle were isolated from a mouse, fixed to a transducer (WPI, FORT100) by using suture (#5-0 silk), and immersed in a 37°C Krebs solution (95%-$O_2$, 5%-$CO_2$ aeration). Electrical stimulation of 40 Hz, 330 ms, and 10V was applied by using two platinum electrodes, and a signal obtained from the transducer was determined as muscle strength (g/mg muscle).

<Results>

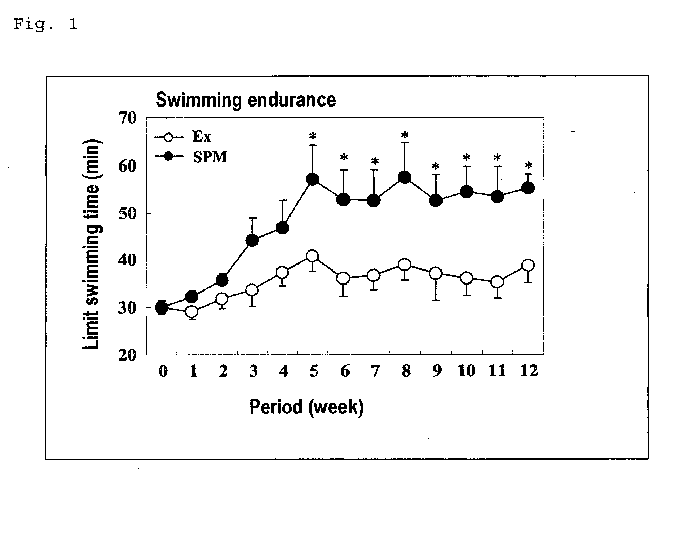

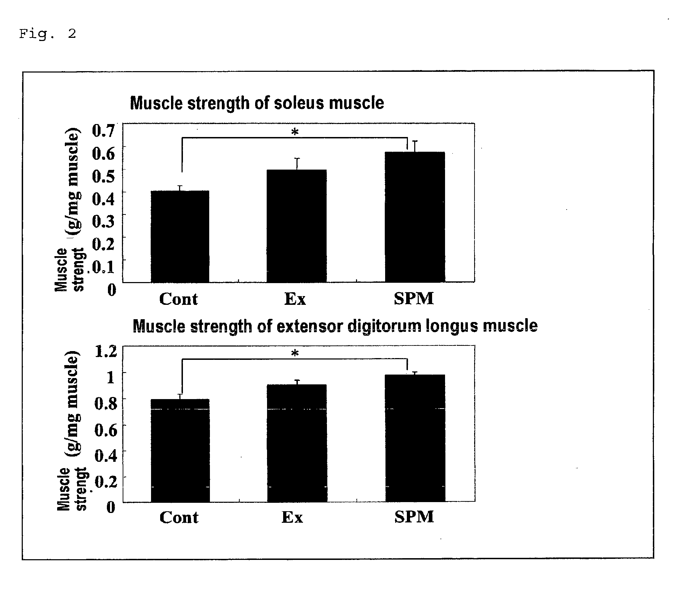

FIG. 1 shows an effect of the sphingomyelin on swimming endurance. In the case of the mice administered with the sphingomyelin, improvement in endurance was observed in the early post-administration period, and significant improvement in endurance was observed five weeks after the beginning of administration compared with the Cont group. Meanwhile, FIG. 2 shows an effect of the sphingomyelin on improvement in muscle strength. The mice administered with the sphingomyelin showed significantly higher values in muscle strength of the soleus muscle and extensor digitorum longus muscle.

The endurance or muscle strength is a representative motor function for taking physical actions. It is thought that improvement in the motor function improves the resistance to physical fatigue. Therefore, in this test, it was revealed that the sphingomyelin was effective for improvement in motor function, endurance, and muscle strength, and anti-fatigue.

Test Example 2: Effect of sphingomyelin on suppression of muscle strength deterioration

<Method>

After preliminary rearing for one week, BALB/c male mice (nine-week old) were classified into three groups (a Normal group, a Cont group, and an SPM group) based on their weights (n = 8 in each group).

After grouping, the mice of the Normal group and the Cont group were fed with a control feed (10% lipid, 20% casein, 55.5% potato starch, 8.1% cellulose, 0.2% methionine, 2.2% vitamin, and 4% mineral), and the mice of the SPM group were fed with a test feed containing the sphingomyelin prepared in Production Example 1 (10% lipid, 20% casein, 55.25% potato starch, 8.1% cellulose, 0.2% methionine, 2.2% vitamin, 4% mineral, and 0.25% sphingomyelin) each for two weeks.

The mice were fed with the test feed for two weeks, and the mice of the Cont group and the SPM group were subject to a tail suspension treatment to exclude a gravity load on their hindlimb muscles (such as soleus muscle). Muscle with a decreased load shows disuse muscle atrophy and has decreased muscle mass and muscle strength. The mice of the Normal group were not subjected to the tail suspension treatment.

After seven days of the tail suspension treatment, the mice were subjected to anatomy, and muscle strength of the isolated soleus muscle was measured. The muscle strength of the isolated muscle was measured in the same way as in Test Example 1.

<Results>

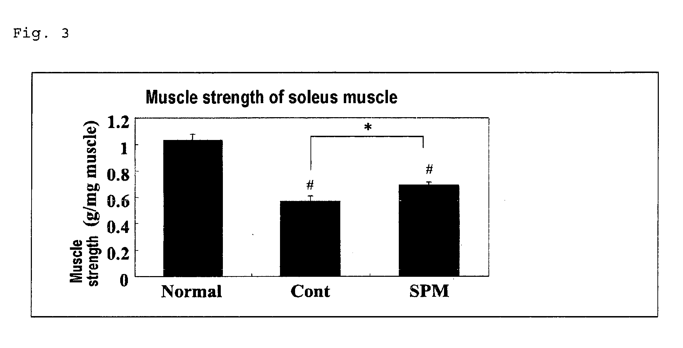

FIG. 3 shows muscle strength of the isolated soleus muscle. The muscle strength of the Cont group and the SPM group significantly decreased by the tail suspension treatment. On the other hand, the muscle strength showed a significantly higher value in the SPM group than in the Cont group. Therefore, the results show that the sphingomyelin has an effect of suppressing muscle strength deterioration.

Test Example 3: Effects of sphingomyelin on promotion of energy consumption and lipid combustion

Actions of the sphingomyelin on promotion of energy consumption and lipid combustion were evaluated as follows. The sphingomyelin was extracted according to the above-mentioned method of Production Example 1 from a milk phospholipid (PC-500, Fonterra Japan).

After preliminary rearing for one week, nine-week old BALB/c mice (male: Oriental Bioservice, Inc.) were classified into two groups (eight mice in each group) such that each group had the same body weight.

After that, the mice were fed with a control feed (10% lipid, 20% casein, 55.5% potato starch, 8.1% cellulose, 0.2% methionine, 2.2% vitamin (product name :Vitamin mix AIN-76, Oriental Bioservice, Inc.), and 4% mineral (product name: Mineral mix AIN-76, Oriental Bioservice, Inc.)) or a test feed containing the sphingomyelin (10% lipid, 20% casein, 54.5% potato starch, 8.1% cellulose, 0.2% methionine, 2.2% vitamin, 4% mineral, and 0.25% sphingomyelin) each for nine weeks, and a respiratory gas analysis was carried out at the tenth week.

The mice were transferred to a chamber for respiratory gas analysis and habituated to the environment for 48 hours, and oxygen consumption level and respiratory quotient of each mouse were measured over 24 hours using Arco-2000 system (ARCOSYSTEM Inc.). As used herein, the oxygen consumption level refers to energy consumption level (mL oxygen consumption level per kg mouse body weight per min (mL/kg/min)), and the respiratory quotient refers to a ratio between carbon-dioxide emission level and oxygen consumption level. From the oxygen consumption level and respiratory quotient, the lipid combustion level was calculated by an equation of Peronnet (Peronnet F, and Massicotte D (1991) Can J Sport Sci 16:23-29.). Table 2 shows average energy consumption level (mL/kg/min) and average lipid combustion level (mg/kg/min) in 24 hours.

After 13 weeks of rearing, the gastrocnemius muscle of a mouse of each group was collected, and RNA samples were obtained using RNeasy Fibrous Tissue Mini Kit (Qiagen). Each RNA sample was quantified, and a reverse transcription reaction was carried out in a reaction solution (1×PCR buffer II (Applied Biosystems), 5 mM MgCl2, 1_mM dNTP mix, 2.5 $\mu$M Oligo d[T]$_{18}$ (New England Biolabs), and 1 U/ml RNase inhibitor (TAKARA BIO INC.)) for 125 ng of RNA per reaction, to thereby obtain cDNA. The reaction was carried out under conditions of 42°C and 10 min, 52°C and 30 min, and 99°C and 5 min.

Quantitative PCR was carried out using the thus obtained cDNA as a template by ABI PRISM 7700 Sequence Detector (Applied Biosystems). The results were corrected based on the expression amount of 36B4 mRNA and represented as relative mRNA expression amounts. 36B4 (GenBank: NM_007475, Forward: GACATCACAGAGCAGGCCCT (SEQ ID NO: 1), Reverse: TCTCCACAGACAATGCCAGG (SEQ ID NO: 2)), PGC-1$\alpha$ (GenBank: NM_008904, Forward: CCGAGAATTCATGGAGCAAT (SEQ ID NO: 3), Reverse: TTTCTGTGGGTTTGGTGTGA (SEQ ID NO: 4)) were used as primers. Table 1 shows the results.

[Table 1]

| Expression of gastrocnemius muscle mitochondrial function-related gene after 13 weeks of rearing | | |
|---|---|---|
| Gene | Control group | Test feed group |
| PGC-1$\alpha$ | 1.17$\pm$0.10 | 1.50$\pm$0.15* |
| Statistically significant difference relative to control group: *$p$<0.05 (t-test) | | |

Table 1 shows that, in the case of the mice fed with the test feed containing the sphingomyelin, PGC-1$\alpha$ gene related to mitochondrial biogenesis and lipid combustion was significantly highly expressed in the gastrocnemius muscle compared with the control feed group. Therefore, the sphingomyelin is useful as a mitochondrial function improver.

[Table 2]

| Average oxygen consumption level in 24 hours after nine weeks of rearing | | |
|---|---|---|
| | Oxygen consumption level (mL/kg/min) | Lipid combustion level (mg/kg/min) |
| Control group | 55.0$\pm$1.0 | 5.4$\pm$0.7 |
| Test feed group | 58.2$\pm$0.8* | 7.5$\pm$0.7* |
| Statistically significant difference relative to control group: *$p$<0.05 (t-test) | | |

Table 2 shows that, in the case of the mice fed with the test feed containing the sphingomyelin, the oxygen consumption level and lipid combustion level were significantly higher than those of the control feed group. Therefore, the sphingomyelin is useful as an energy consumption promoter and a lipid combustion promoter.

Preparation Example

Formulation Example 1: Motor function improving, mitochondrial function improving, or energy consumption promoting jelly food

A 0.65% mixed gelling agent of carrageenan and Locust bean gum, 5.0% concentrated fruit juice of 50% grapefruit, 0.05% citric acid, 0.05% vitamin C, and a 0.1% sphingomyelin (NM-70 produced by NOF CORPORATION) are mixed. Water is added to the mixture so as to adjust to 100%, and the mixture is dissolved at 65°C. Furthermore, to the mixture solution, a small amount of a grape fruit flavor is added, and the mixture solution is held for five minutes at 85°C to carry out sterilization. After that, the mixture solution is dispensed into 100 mL vessels. The mixture is allowed to stand for eight hours while it is gradually cooled to 5°C and gelled to obtain a jelly food containing the sphingomyelin and having good solubility in the mouth, fruit flavor, and good texture.

Formulation Example 2: Motor function improving, mitochondrial function improving, or energy consumption promoting tablet

A tablet is produced by formulation (daily dosage: 2200 mg) composed of 180 mg of ascorbic acid, 50 mg of citric acid, 12 mg of aspartame, 24 mg of magnesium stearate, 120 mg of crystalline cellulose, 594 mg of lactose, and 120 mg of a sphingomyelin (NM-10 produced by NOF CORPORATION) according to Japanese Pharmacopoeia (General Rules for Preparation: "Tablets"). Thus, tablets containing the sphingomyelin are obtained.

Formulation Example 3: Motor function improving, mitochondrial function improving, or energy consumption promoting Vitamin oral liquid

To an appropriate amount of purified water, 800 mg of taurine, 2000 mg of sucrose, 50 mg of caramel, 30 mg of sodium benzoate, 5 mg of vitamin B1 nitrate, 20 mg of vitamin B2, 20 mg of vitamin B6, 2000 mg of vitamin C, 100 mg of vitamin E, 2000 IU of vitamin D3, 20 mg of nicotinamide, 50 mg of the purified sphingomyelin (Production Example 1), 200 mg of leucine, 100 mg of isoleucine, and 100 mg of valine are added and dissolved. The mixture solution is adjusted to pH 3 with an aqueous solution of phosphoric acid. Purified water is further added so that the total amount became 50 mL. The resultant is sterilized at 80°C for 30 minutes to obtain a motor function improving, mitochondrial function improving, or energy consumption promoting beverage containing the sphingomyelin and amino acids.

Formulation Example 4: Motor function improving, mitochondrial function improving, or energy consumption promoting milk beverage

Purified water is added to 3.4 g of milk casein, 1.67 g of isolated soybean protein, 14.86 g of dextrin, 1.3 g of sucrose, 1.75 g of soybean oil, 0.18 g of Perilla oil, 0.14 g of soybean phospholipid, 0.07 g of glycerin fatty acid ester, 0.60 g of minerals, 0.06 g of vitamins, and 100 mg of the purified sphingomyelin (Production Example 1). The mixture is subjected to retort sterilization according to an ordinary method to obtain a motor function improving, mitochondrial function improving, or energy consumption promoting beverage (100 mL) containing the sphingomyelin.

## Claims

1.  A sphingomyelin for use in a method for suppressing muscle strength deterioration.

2.  The sphingomyelin of claim 1 for use in a method for treating or preventing locomotive syndrome.

3.  The sphingomyelein of claim 1 or 2 for use in a method for treating or preventing sarcopenia.

## Patentansprüche

1.  Sphingomyelin zur Verwendung in einem Verfahren zur Unterdrückung des Muskelkraftabbaus.

2.  Sphingomyelin nach Anspruch 1 zur Verwendung in einem Verfahren zur Behandlung oder Prävention des Locomotive-Syndroms.

3. Sphingomyelin nach Anspruch 1 oder 2 zur Verwendung in einem Verfahren zur Behandlung oder Prävention von Sarkopenie.

**Revendications**

1. Sphingomyéline pour son utilisation dans une méthode de suppression de la détérioration de la force musculaire.

2. Sphingomyéline selon la revendication 1 pour son utilisation dans une méthode de traitement ou de prévention d'un syndrome locomoteur.

3. Sphingomyéline selon la revendication 1 ou 2 pour son utilisation dans une méthode de traitement ou de prévention de la sarcopénie.

Fig. 1

Fig. 2

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002226394 A
- JP 2000350563 A
- WO 2008081934 A
- US 2009253658 A
- US 2004043013 A
- JP 2002065212 A
- JP 2005089384 A
- WO 2005074962 A
- JP 2009107987 A
- JP 2004149494 A
- JP 2007314446 A

- JP 2008063318 A
- JP 2004067629 A
- JP 2002322058 A
- JP 2000250563 A
- JP 2007246404 A
- JP 2007112793 A
- JP 11269074 A
- JP 2003252765 A
- EP 11739761 A
- JP 2010022221 A
- JP 2010022223 A

**Non-patent literature cited in the description**

- **DANIELI-BETTO et al.** *American Journal of Physiology - Cell Physiology,* vol. 288 (6), C1367-C1373
- **ANDERSON.** JAMA. vol. 223
- **VON ALLWORDEN.** Phospholipids. AOCS Press, 1995
- **KINGSLEY.** *Med Sci Sports Exerc,* vol. 38
- **IWANAMIKOUZA.** *Gendaiigaku no kiso,* 1999, vol. 12 (2), 55-58
- **RITZ P.** *Diabetes Metab.,* 2005, vol. 2, 5S67-5S73
- **HOLLOSZY JO.** *J. Physiol. Pharmacol.,* 2008, vol. 59, 5-18
- **DULLOO AG.** *Am J Clin Nutr.,* 1989, vol. 49 (1), 44-50
- **KAWADA T.** *Proc Soc Exp Biol Med.,* 1986, vol. 183 (2), 250-6

- **OHNUKI K.** *Biosci Biotechnol Biochem.,* 2001, vol. 65 (12), 2735-40
- **NAGATA Y.** *J Histochem Cytochem.,* 2006, vol. 54 (4), 375-384
- **FURUYA H.** *Int J Vitam Nutr Res.,* 2008, vol. 78 (1), 41-49
- **WEIS.** *Chem Phys Lip,* 1999, vol. 3
- **DONG.** *Tetrahedron Lett,* 1991, vol. 5291
- **BYUN.** *J Org Chem,* 1994, vol. 6495
- **SANCHEZ-JUANES.** *Int Dairy J,* 2009, vol. 273
- **PERONNET et al.** *Can J Sport Sci.,* 1991, vol. 16, 23-29
- **MATSUMOTO.** *J Appl Physiol.,* 1996, vol. 81, 1843-1849
- **CANNON et al.** *Biomed Sci Instrum,* 2005
- **PERONNET F ; MASSICOTTE D.** *Can J Sport Sci,* 1991, vol. 16, 23-29